# EUROPÄISCHE PATENTANMELDUNG

(11) **EP 1 293 167 A2**
(43) Veröffentlichungstag der Anmeldung: **19.03.2003**
(21) Anmeldenummer: 02020692.6
(22) Anmeldetag: 13.09.2002
(51) Int. Cl.: A61B 17/32

(54) **Vorrichtung zur Durchtrennung und Entnahme von Gewebe**

(30) Priorität: 14.09.2001 EP 01122131
(71) Anmelder: TuiLaser AG, 82110 Germering (DE)
(72) Erfinder: Hohla, Alexander, 80798 München (DE); Leipert, Gunther, 82205 Gilching (DE)
(74) Vertreter: Hofstetter, Alfons J., Dr.rer.nat.

(57) **Zusammenfassung**

Die vorliegende Erfindung betrifft eine Vorrichtung zur Durchtrennung und Entnahme von menschlichem oder tierischem Gewebe oder Gewebemembranen mit einer als Hohlnadel ausgebildeten Schneidevorrichtung 12, wobei die Schneidevorrichtung 12 an ihrem kreisförmigen distalen Ende 14 über zumindest einen Teilbereich mindestens eine Schneidkante 16 aufweist. Die Schneidevorrichtung 12 besteht dabei aus mindestens einem kreis- oder röhrenförmigen ersten, inneren Element 20, welches die Schneidkante 16 aufweist und eine kreisförmige Schnittfläche 26 ausbildet, wobei innerhalb der Schneidevorrichtung 12 mindestens ein Schneidelement 28 zur Durchführung eines Schnitts senkrecht zur Eindringrichtung der Schneidevorrichtung 12 und parallel zur Schnittfläche 26 in einem vorbestimmten Abstand zum distalen Ende der Schneidevorrichtung 12 angeordnet ist.

## Beschreibung

Die vorliegende Erfindung bezieht sich auf eine Vorrichtung zur Durchtrennung und Entnahme von menschlichem oder tierischem Gewebe oder Gewebemembranen mit einer als Hohlnadel ausgebildeten Schneidevorrichtung, wobei die Schneidevorrichtung an ihrem kreisförmigen distalen Ende über zumindest einen Teilbereich mindestens eine Schneidkante aufweist.

Derartige mechanische Schneidevorrichtungen zur Durchtrennung von menschlichen oder tierischen Gewebemembranen sind in den Patentschriften US-A-3 993 079, US-A-5 078 688 und US-A-5 997 486 vorbeschrieben. Sie werden unter anderem zur Behebung von Ventilationsstörungen des Mittelohres, einem der häufigsten Krankheitsbilder in der Hals-, Nasen- und Ohrenheilkunde, eingesetzt. Dabei wird als wichtigste Therapieform dieser Erkrankung die Wiederherstellung der Belüftung der Paukenhöhle durch eine Parazentese mit oder ohne Einlage eines Paukenröhrchens (vgl. DE 85 18 118 U1) angesehen.

Des weiteren sind mechanische Schneidevorrichtungen zur Durchtrennung und Entnahme von menschlichem oder tierischem Gewebe bekannt. So beschreibt de US-A-6 155 989 eine Vorrichtung zur Entnahme von Gewebeproben die eine röhrenförmige Schneidevorrichtung aufweist. In der Schneidevorrichtung ist zudem eine Vakuumeinrichtung angeordnet, welche zur Fixierung und Entnahme der entsprechenden Gewebeproben dient.

Diese bekannten mechanischen Schneidevorrichtungen zur Durchtrennung und/oder Entnahme von menschlichen oder tierischen Gewebe oder Gewebemembranen weisen verschiedenartigste Nachteile auf.

So können mit den bekannten Schneidevorrichtungen nur sehr begrenzt Gewebeproben oder Gewebemembranperforationen mit vordefinierten und konstanten Durchmesser hergestellt werden, da aufgrund der Flexibilität und der zum Teil unebenen Oberfläche des Gewebes oder der Gewebemembranen kein definierter Schnitt bzw. eine definierte Perforation angesetzt werden kann. Insbesondere wird bei den bekannten Schneidevorrichtungen lediglich ein Schnitt in die Tiefe des Gewebes oder der Gewebemembran durchgeführt. Die endgültige Abtrennung des Gewebestücks aus dem Gewebeverband wird mittels eines Skalpells, wie dies in der US-A-6 155 989 beschrieben ist, durch eine sogenannte Knipsbiopsie, mittels einer zusätzliche Elektroschlinge oder einfaches Ausreißen des Gewebes durchgeführt. Nachteilig an diesen Verfahren ist jedoch, daß die bekannten Arten der Abtrennung der zu entnehmenden Gewebestücke nicht geeignet sind, Gewebestücke mit konstanten und vordefinierten Abmessungen zu erhalten. Vielmehr sind die senkrecht zur Eindringtiefe der bekannten Schneidevorrichtungen erhaltenen Schnittkanten der Gewebestücke unscharf, weisen Risse auf und können somit nicht mit der eigentlichen Eindringtiefe der Schneidevorrichtung korreliert werden.

Es ist daher Aufgabe der vorliegenden Erfindung, eine gattungsgemäße Vorrichtung zur Durchtrennung und Entnahme von menschlichem oder tierischem Gewebe oder Gewebemembranen bereitzustellen, welche die Entnahme eines Gewebestücks mit konstanten, vordefinierten Abmessungen gewährleistet.

Gelöst wird diese Aufgabe durch eine gattungsgemäßen Vorrichtung mit den Merkmalen des Anspruches 1.

Vorteilhafte Ausgestaltungen sind in den Unteransprüchen beschrieben.

Bei einer erfindungsgemäßen Vorrichtung zur Durchtrennung und Entnahme von menschlichem oder tierischem Gewebe oder Gewebemembranen besteht die Schneidevorrichtung aus mindestens einem kreis- oder röhrenförmigen ersten, inneren Element, welches eine Schneidkante aufweist und eine kreisförmige Schnittfläche ausbildet, wobei innerhalb der Schneidevorrichtung mindestens ein Schneidelement zur Durchführung eines Schnitts senkrecht zur Eindringrichtung der Schneidevorrichtung und parallel zur Schnittfläche in einem vorbestimmten Abstand zum distalen Ende der Schneidevorrichtung angeordnet ist. Durch die Ausbildung einer definierten kreisförmigen Schnittfläche durch das kreis- oder röhrenförmige erste innere Element sowie durch die Anordnung mindestens eines Schneidelements innerhalb der Schneidevorrichtung 12 ist gewährleistet, dass nicht nur parallel zur Eindringtiefe der Schneidevorrichtung, sondern auch senkrecht dazu ein exakter Schnitt ausgeführt werden kann. Durch diese Schnittführung ist es möglich, Gewebestücke mit konstanten und vordefinierten Abmessungen zu erhalten. Derartige exakt in ihrer Geometrie definierte Gewebestücke ermöglichen es zum Beispiel in der Histologie, ein eindeutiges Tiefenprofil der Gewebeschichtung zu erstellen. Derartiges ist bei den vorbekannten Vorrichtungen und Verfahren, wie oben beschrieben, nicht möglich. Insbesondere kann die Schneidevorrichtung aus zwei zueinander beweglichen kreisförmigen oder röhrenförmigen Elementen mit einer gemeinsamen Drehachse bestehen. Damit ist es möglich, durch ein einfaches Verdrehen der Elemente zueinander sowohl einen senkrechten wie auch einen horizontalen Gewebeschnitt durchzuführen.

In einer vorteilhaften Ausgestaltung der Erfindung ist das Schneidelement ein Schneiddraht und eine drahtähnliche Schneide. Dabei ist es einerseits möglich, dass mindestens ein Schneiddraht oder mindestens eine drahtähnliche Schneide innerhalb des ersten, inneren Elements entlang des inneren Durchmessers des ersten Elements angeordnet ist. Es ist aber auch möglich, dass an dem ersten, inneren Element ein erstes Ende des Schneiddrahtes oder der drahtähnlichen Schneide angeordnet ist, wobei ein dem ersten Ende gegenüberliegendes zweites Ende des Schneiddrahts oder der drahtähnlichen Schneide mit dem zweiten äußeren Element verbunden ist, derart, dass bei einem Verdrehen der beiden Elemente zueinander der Schneiddraht oder die drahtähnliche Schneide, die durch das innere, erste Element gebildete Schnittfläche der Schneidevorrichtung in vorbestimmtem Abstand überfährt. Beide Anordnungen erlauben eine exakte Schnittführung senkrecht zur Eindringrichtung der Schneidevorrichtung.

In einer weiteren vorteilhaften Ausgestaltung der Erfindung besteht das Schneidelement aus mindestens einer schwenkbar innerhalb des ersten, inneren Elements gelagerten und angeordneten Schneide, wobei beim Verdrehen des Elements die Schneide, die durch das innere erste Element gebildete Schnittfläche der Schneidevorrichtung in vorbestimmtem Abstand überfährt. Vorteilhafterweise ist dabei die Schneide gebogen ausgebildet. Durch ein Schwenken der Schneide in das Innere des ersten Elements ergibt sich ebenfalls eine exakte Schnittführung senkrecht zur Eindringtiefe der Schneidevorrichtung. Es ist aber auch möglich, dass mehrere schwenkbare Schneiden ausgebildet sind, die über eine Mechanik von außen nach innen geführt werden. In diesem Fall muss das erste Element beziehungsweise die Schneidevorrichtung nicht gedreht werden.

Bei der Ausbildung des Schneidelements als Schneiddraht kann mindestens ein Ende des Schneiddrahts über eine Ausgleichsfeder oder eine Ausgleichsrolle mit dem ersten oder zweiten Element verbunden sein. Dadurch ist gewährleistet, dass der Schneiddraht immer straff gespannt ist, so dass sich eine exakte Schnittlinie ergibt.

In einer weiteren vorteilhaften Ausgestaltung der Erfindung weist mindestens ein Schneidelement eine Ultraschallanbindung auf. Damit kann zuverlässig verhindert werden, dass Gewebe an dem Schneidelement oder innerhalb der Schneidevorrichtung hängen bleibt. Zudem ergibt sich eine bessere Schneidwirkung.

In einer weiteren vorteilhaften Ausgestaltung der Erfindung ist mindestens ein Schneidelement mit Strom und/oder Hochfrequenz beaufschlagt. Durch die entstehende Wärme werden die Schnittflächen am Gewebe vorteilhafterweise verödet und eventuelle Blutungen gestillt.

In einer weiteren vorteilhaften Ausgestaltung der Erfindung bestehen das oder die Schneidelemente aus einem lichtdurchlässigen Material. Damit ist es vorteilhafterweise möglich, Licht, zum Beispiel Laserlicht, in das Innere der Schneidvorrichtung beziehungsweise das Innere des ersten Elements einzukoppeln, wodurch zum Beispiel eine zusätzliche Koagulation nach der Biopsieentnahme möglich ist, so dass entsprechende Blutungen gestoppt werden können.

In einer weiteren vorteilhaften Ausgestaltung der Erfindung ist innerhalb des ersten hohlförmigen Elements eine Fasersonde zur Einkopplung eines Lasers, für spektroskopische Messungen, für die optische Kohärenztomographie oder für die konfokale Mikroskopie angeordnet. Durch derartige Maßnahmen wird das Anwendungsgebiet der hier beschriebenen Vorrichtung zur Durchtrennung und Entnahme von Gewebe deutlich erhöht.

In einer vorteilhaften Ausgestaltung der Erfindung ist innerhalb einer hohlförmigen Schneidevorrichtung ein erster Unterdruckkanal zum Ansaugen und Fixieren des zu durchtrennenden und zu entnehmenden Gewebes ausgebildet. Durch das Ansaugen und Fixieren des Gewebestücks vor oder während des Schneidevorgangs ist gewährleistet, dass eine definierte Gewebeoberfläche, die dem Durchmesser der Schneidevorrichtung entspricht, ausgeschnitten werden kann. So ist es vorteilhafterweise möglich, dass mit einem einfachen Dreh- und Schneidemechanismus je nach Durchmesser des Gerätes problemlos runde Löcher mit konstantem Durchmesser in das Gewebe oder in die Gewebemembran, wie z.B. in das Trommelfell geschnitten werden können. Zusätzlich kann durch das Ansaugen auch eine stark retrahierte Gewebemembran bzw. ein stark retrahiertes Trommelfell von einer Unterlage gelöst werden, was vorteilhafterweise in einer geringen Verletzungsgefahr für Strukturen unterhalb der Membran wie z.B. im Mittelohr mit Hammer und Steigbügel im Vergleich zu konventionellen Methoden mit bekannten Schneidevorrichtungen resultiert. Selbst bei einem sich in Lokalanästhesie bewegendem Patienten ist ein präziser Schneidevorgang gewährleistet. Die erfindungsgemäße Vorrichtung muss lediglich an eine herkömmlichen Vakuumvorrichtung angeschlossen werden, die in jeder Praxis und jedem Operationssaal eines Krankenhauses vorhanden ist. Aufwendige Geräte wie z.B. Laser sind nicht notwendig. Zudem kann aufgrund der Saugwirkung der erfindungsgemäßen Vorrichtung während bzw. nach der Gewebeentnahme oder dem Perforationsvorgang die von der Schneidevorrichtung abgetrennten Gewebeteile und/oder Sekret aus dem Bereich unterhalb/hinter der Gewebemembran wie z.B. dem Mittelohr abgesaugt werden ohne dass die Vorrichtung in der Hand gewechselt werden muss. Dies ermöglicht vorteilhafterweise einen leichteren und schnelleren Operationsvorgang. Gewebeteile können festgehalten werden und zusammen mit der Vorrichtung entfernt werden.

In einer vorteilhaften Ausgestaltung der erfindungsgemäßen Vorrichtung ist die Schneidevorrichtung von einem zweiten Unterdruckkanal zum Ansaugen und Fixieren des zu durchtrennenden Gewebes umgeben. Dadurch ist eine zusätzliche Fixierung des Gewebes oder der Gewebemembran gewährleistet.

In einer weiteren vorteilhaften Ausgestaltung der Erfindung ist der Unterdruck im ersten und/oder zweiten Unterdruckkanal mittels einer Vakuumvorrichtung voreinstellbar und/oder regulierbar. Durch die Einstellbarkeit und Regulierbarkeit des auf die Gewebeoberfläche oder die Gewebemembran wirkenden Unterdrucks ist gewährleistet, dass ungewollte Schädigungen des Gewebes durch eine zu hohe Druckeinwirkung vermieden werden. Die beiden Unterdruckkanäle können dabei abhängig oder unabhängig voneinander reguliert werden.

In einer weiteren vorteilhaften Ausgestaltung der Vorrichtung ist im ersten Unterdruckkanal mindestens ein Sieb oder mindestens eine Filtervorrichtung angeordnet. So können die abgetrennten Gewebestücke mit der Vorrichtung herausgenommen und die Schnittkanten analysiert werden.

Weitere Einzelheiten, Vorteile und Merkmale der vorliegenden Erfindung ergeben sich aus den folgenden, in den Figuren dargestellten Ausführungsbeispielen. Es zeigen
- Figur 1: eine schematische Darstellung einer erfindungsgemäßen Vorrichtung zur Durchtrennung und Entnahme von menschlichem oder tierischem Gewebe oder Gewebemembranen;
- Figur 2: eine schematische Darstellung eines Schneidelements der erfindungsgemäßen Vorrichtung gemäß einer ersten Ausführungsform; und
- Figur 3: eine schematische Darstellung eines Schneidelements der erfindungsgemäßen Vorrichtung gemäß einer zweiten Ausführungsform.

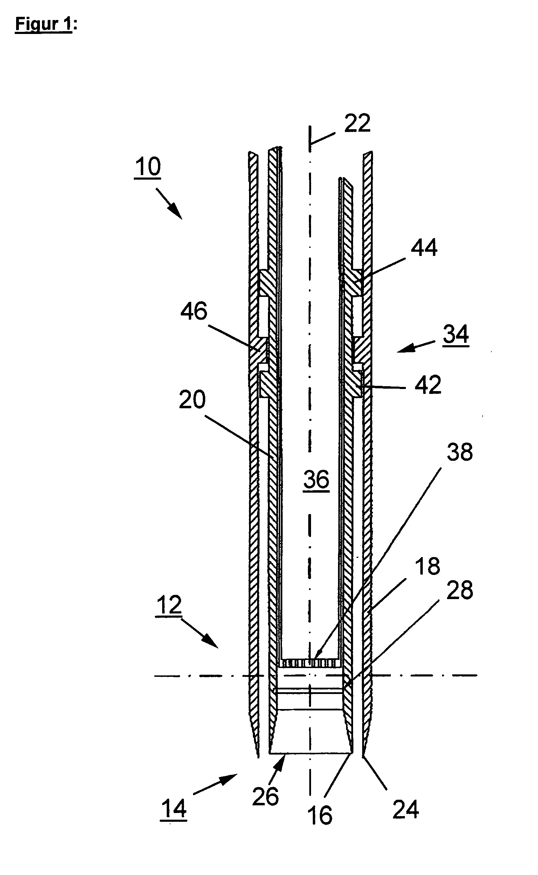

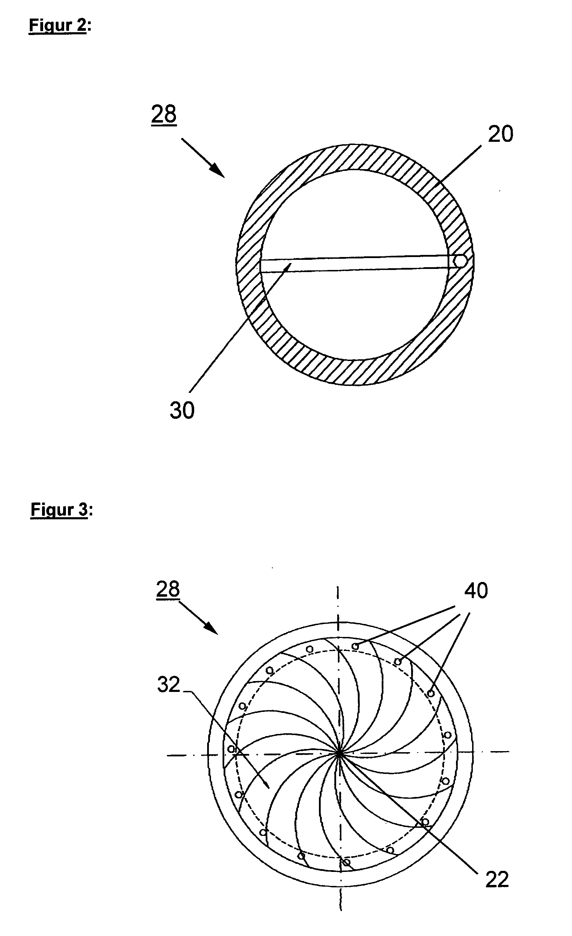

Figur 1 zeigt in einer schematischen Darstellung eine Vorrichtung 10 zur Durchtrennung und Entnahme von menschlichem oder tierischem Gewerbe oder Gewebemembranen mit einer als Hohlnadel ausgebildeten Schneidevorrichtung 12. Die Schneidevorrichtung 12 weist dabei an ihrem kreisförmigen distalen Ende 14 zwei Schneidkanten 16, 24 auf. Man erkennt zudem, dass die in dem Ausführungsbeispiel dargestellte Schneidevorrichtung 12 aus zwei zueinander beweglichen kreisförmigen und röhrenförmigen Elementen 18, 20 mit einer gemeinsamen Drehachse 22 besteht. Beide Elemente 18, 20 weisen dabei jeweils eine Schneidkante 16, 24 auf. Des weiteren erkennt man, dass das erste, innere Element 20 an seinem distalen Ende zwischen den Schneidkanten 16 eine Schneidfläche 26 ausbildet. Innerhalb der Schneidevorrichtung 12 beziehungsweise innerhalb des ersten Elements 20 ist in einem vorbestimmten Abstand zum distalen Ende 14 der Schneidevorrichtung 12 ein Schneidelement 28 zur Durchführung eines Schnitts senkrecht zur Eindringrichtung der Schneidevorrichtung 12 und parallel zur Schnittfläche 26 angeordnet. Das Schneideelement 28 kann dabei als Schneiddraht 30, als drahtähnliche Schneide 30 oder als Schneide 32 ausgebildet sein (vergleiche Figuren 2 und 3).

In dem dargestellten Ausführungsbeispiel ist ein Schneidelement 28 innerhalb des ersten, inneren Elements 20 entlang des inneren Durchmessers des ersten Elements 20 angeordnet. Es ist aber auch möglich, dass an dem ersten, inneren Element 20 ein erstes Ende des Schneidelements 28 beziehungsweise Schneiddrahts 30 angeordnet ist, wobei ein dem ersten Ende gegenüberliegendes zweites Ende des Schneidelements 28 beziehungsweise des Schneiddrahts 30 mit dem zweiten, äußeren Element 18 verbunden ist, derart, dass bei einem Verdrehen der beiden Elemente 18, 20 zueinander das Schneidelement 28 beziehungsweise der Schneiddraht die durch das innere erste Element 20 gebildete Schnittfläche 26 der Schneidevorrichtung 12 in vorbestimmtem Abstand überfährt (nicht dargestellt).

Des weiteren erkennt man, dass die Eindringtiefe der Schneidevorrichtung 12 mittels einer Einstellvorrichtung 34 einstellbar ist. Hierzu weist das erste innere Element 20 in einem vom distalen Ende 14 der Schneidevorrichtung 12 definierten Abstand an ihrem äußeren Umfang zwei Vorsprünge 42, 44 auf, die einen entsprechenden Anschlag 46, der am Innenumfang des zweiten, äußeren Elements 18 angeordnet ist, aufnehmen, so dass es zu einer Begrenzung des Vorschubs und damit der Eindringtiefe des ersten Elements 20 kommt.

Innerhalb des ersten hohlförmigen Elements 20 ist zudem ein erster Unterdruckkanal 36 zum Ansaugen und Fixieren des zu durchtrennenden und zu entnehmenden Gewebes ausgebildet. Die Schneidevorrichtung 12 kann aber auch von einem zweiten Unterdruckkanal zum Ansaugen und Fixieren des zu durchtrennenden Gewebes umgeben sein (nicht dargestellt). Der Unterdruck im ersten und/oder zweiten Unterdruckkanal 36 ist dabei mittels einer Vakuumvorrichtung (nicht dargestellt) voreinstellbar und regulierbar. Innerhalb des ersten Unterdruckkanals 36 ist ein Sieb 38 angeordnet. Durch das Sieb 38 wird das entnommene Gewebestück rückseitig durch Unterdruck festgehalten. Über einen (nicht dargestellten) Stößel kann das Gewebestück dann aus dem Sieb 38 und des Schneidevorrichtung 12 entnommen werden.

Figur 2 zeigt eine schematische Darstellung des Schneidelements 30 der Vorrichtung 10 gemäß einer ersten Ausführungsform. Man erkennt, dass das Schneidelement 28 aus einem Schneiddraht 30 besteht. Der Schneiddraht 30 ist dabei innerhalb des ersten hohlförmigen Elements 20 entlang des inneren Durchmessers des ersten Elements 20 angeordnet.

Figur 3 zeigt eine schematische Darstellung eines aus Schneide 32 ausgebildeten Schneidelements 28 der Vorrichtung 10 gemäß einer zweiten Ausführungsform. Man erkennt, dass mehrere Schneiden 32 über eine Mechanik von außen nach innen geführt werden und somit einen Schnitt parallel zur Schnittfläche 26 erlauben. Jedes der Schneidelemente 32 ist dabei schwenkbar am oder innerhalb des ersten, inneren Elements 20 über eine Drehverbindung 40 gelagert. Die Schneiden 32 schließen sich dabei in Richtung der Drehachse 22 und führen somit den genannten Schnitt senkrecht zur Eindringtiefe der Schneidevorrichtung 12 durch. Vorteilhafterweise bilden die geschlossenen Schneiden 32 eine Art Boden einer Probenkammer aus, so dass die Entnahme der Gewebeprobe zusammen mit der Vorrichtung 10 möglich ist. Man erkennt, dass die Schneiden 32 gebogen ausgebildet sind.

Des weiteren ist es möglich, dass mindestens ein Schneidelement 28, 30, 32 eine Ultraschallanbindung aufweist oder mit Strom und/oder Hochfrequenz beaufschlagt ist. Des weiteren können die Schneidelemente 28, 30, 32 aus einem lichtdurchlässigen Material bestehen. Die Schneidkanten 16, 24 wie auch die Schneidelemente 28, 30, 32 können mit Diamant verstärkt sein, so dass die Schneidkanten sehr hart und scharf ausgebildet sind.

Die Vorrichtung 10 eignet sich ganz allgemein zur Entnahme von Biopsien. Insbesondere können damit verdächtige Muttermale, flache Tumorläsionen oder Ähnliches punktiert werden. Auf die Biopsieentnahme bei der Stereotaxie, insbesondere in Bereichen mit härterem Gehirngewebe ist möglich. Des weiteren besteht die Möglichkeit, die Vorrichtung 10 als Mikrokeratom zur Entfernung der obersten Hornhautschicht bei entsprechenden chirurgischen Eingriffen zur Korrektur von Sehschwächen zu verwenden.

Die Vorrichtung 10 kann auch als Katheter ausgebildet sein. Dies bedeutet, dass die Vorrichtung 10 bzw. deren Bestandteile aus flexiblen Materialien bestehen. Geeignete Materialien sind z.B. biostabile Kunststoffe. Es ist aber auch möglich, die Vorrichtung 10 relativ starr auszubilden. In diesem Fall bestehen die Einzelelemente der Vorrichtung 10 aus Kunststoff, Metall oder einer Metall-Legierung. Zudem ist es möglich, daß die Vorrichtung 10 formbar ausgebildet ist, derart, daß sie an die anatomischen Gegebenheiten von Körperhöhlen z.B. des Ohres angepasst werden kann.

Im Anwendungsfall wird die Vorrichtung 10 mit dem distalen Ende der Schneidevorrichtung 12 auf das zu durchtrennende und zu entnehmende Gewebe aufgesetzt. Der oder die Unterdruckkanal/Unterdruckkanäle 36 saugen die Gewebeoberfläche 20 an und fixieren diese an dem distalen Ende der Vorrichtung 10. Durch ein leichtes Drehen der Vorrichtung 10 bzw. der Schneidevorrichtung 12 nach dem Aufsetzten wird das Gewebe perforiert und die Schneidevorrichtung dringt in das Gewebe ein. Die Eindringtiefe der Schneidevorrichtung wird dabei durch die Einstellvorrichtung 34 begrenzt. Es ist aber auch möglich, die Höhe des Unterdrucks derart zu wählen, dass durch die Druckeinwirkung auf das Gewebe dieses derart an die Schneidkanten 16, 24 der Schneidevorrichtung 12 gedrückt wird, dass auch ohne eine zusätzliche mechanische Bewegung der Vorrichtung 10 eine Perforation erfolgt. Auch andere Schneidemechanismen wie z.B. eine Perforation durch Vibration der Vorrichtung 10 oder Kombinationen verschiedener Schneidmechanismen sind denkbar. Nach Erreichen der vorbestimmten Eindringtiefe erfolgt entweder ein Drehen des ersten Elements 20 oder ein Verdrehen des ersten und zweiten Elements 18, 20 zueinander, so daß der Schneiddraht 30 die durch das innere erste Element 20 gebildete Schnittfläche 26 der Schneidevorrichtung 12 in vorbestimmten Abstand überfährt oder die schwenkbar innerhalb des ersten Elements 20 angeordnete Schneide 32 überfährt beim Drehen des ersten Elements 20 oder bei dem Verdrehen der beiden Elemente 18, 20 zueinander die durch das innere erste Element 20 gebildete Schnittfläche 26 der Schneidevorrichtung 12 in vorbestimmten Abstand. Es ist aber auch möglich, daß mehrere Schneiden 32-ohne ein Drehen des ersten Elements 20 oder ohne ein Verdrehen der beiden Elemente 18, 20 zueinander - über eine Mechanik von Außen nach Innen geführt werden.

Die Schneidevorrichtung 12 kann zudem eine Fixiervorrichtung, wie z.B. Ballons oder Federn, aufweisen (nicht dargestellt), welche die Schneidevorrichtung 12 in einer Körperhöhle fixiert und festhält.

Weitere, nicht dargestellte Ausführungsformen der Vorrichtung 10 sind möglich. So kann eine Anpassung des distalen Endes der Vorrichtung 10 an die Anatomie der Körperhöhle, in welche die Vorrichtung 10 eingeführt wird erfolgen. So ist beispielsweise das Trommelfell trichterförmig eingezogen und liegt zudem schräg im Gehörgang. Des weiteren ist es möglich, daß ein weiterer Zugang zur Einführung einer Sensorik oder Bildleitern zur visuellen Inspektion des Schneidvorganges oder der Schnittkanten ausgebildet ist. Es ist zudem denkbar, daß eine Feder oder Druck als Impulsantrieb für die Schneidvorrichtung 12 verwendet wird.

## Patentansprüche

1. Vorrichtung zur Durchtrennung und Entnahme von menschlichem oder tierischem Gewebe oder Gewebemembranen mit einer als Hohlnadel ausgebildeten Schneidevorrichtung (12), wobei die Schneidevorrichtung (12) an ihrem kreisförmigen distalen Ende (14) über zumindest einen Teilbereich mindestens eine Schneidkante (16) aufweist,
**dadurch gekennzeichnet,**
**daß** die Schneidevorrichtung (12) aus mindestens einem kreis- oder röhrenförmigen ersten, inneren Element (20), welches die Schneidkante (16) aufweist und eine kreisförmige Schnittfläche (26) ausbildet, besteht, wobei innerhalb der Schneidevorrichtung (12) mindestens ein Schneidelement (28) zur Durchführung eines Schnitts senkrecht zur Eindringrichtung der Schneidevorrichtung (12) und parallel zur Schnittfläche (26) in einem vorbestimmten Abstand zum distalen Ende der Schneidevorrichtung (12) angeordnet ist.

2. Vorrichtung nach Anspruch 1,
**dadurch gekennzeichnet,**
**daß** die Schneidevorrichtung (12) aus zwei zueinander beweglichen kreisförmigen oder röhrenförmigen Elementen (18, 20) mit einer gemeinsamen Drehachse (22) besteht.

3. Vorrichtung nach Anspruch 1 oder 2,
**dadurch gekennzeichnet,**
**daß** das Schneidelement (28) ein Schneiddraht (30) oder eine drahtähnliche Schneide (30) ist.

4. Vorrichtung nach Anspruch 3,
**dadurch gekennzeichnet,**
**daß** an dem ersten, inneren Element (20) ein erstes Ende des Schneiddrahtes (30) oder der drahtähnlichen Schneide (30) angeordnet ist, wobei ein dem ersten Ende gegenüberliegendes zweites Ende des Schneiddrahts (30) oder der drahtähnlichen Schneide (30) mit dem zweiten, äußeren Element (18) verbunden ist, derart, dass bei einem Verdrehen der beiden Elemente (18, 20) zueinander der Schneiddraht (30) oder die drahtähnliche Schneide (30) die durch das innere erste Element (20) gebildete Schnittfläche (26) der Schneidevorrichtung (12) in vorbestimmten Abstand überfährt.

5. Vorrichtung nach Anspruch 3,
**dadurch gekennzeichnet,**
**daß** mindestens ein Schneiddraht (30) oder mindestens eine drahtähnliche Schneide (30) innerhalb des ersten, inneren Elements (20) entlang des inneren Durchmessers des ersten Elements (20) angeordnet ist.

6. Vorrichtung nach Anspruch 1 oder 2,
**dadurch gekennzeichnet,**
**daß** das Schneidelement (28) aus mindestens einer schwenkbar innerhalb des ersten, inneren Elements (20) gelagerten und angeordneten Schneide (32) besteht, wobei bei einem Verdrehen des Elements (20) die Schneide (32) die durch das innere erste Element (20) gebildete Schnittfläche (26) der Schneidevorrichtung (12) in vorbestimmten Abstand überfährt.

7. Vorrichtung nach Anspruch 6,
**dadurch gekennzeichnet,**
**daß** die Schneide (32) gebogen ausgebildet ist.

8. Vorrichtung nach Anspruch 6 oder 7,
**dadurch gekennzeichnet,**
**daß** mehrere Schneiden (32) über eine Mechanik von Außen nach Innen geführt werden.

9. Vorrichtung nach einem der Ansprüche 2 bis 4,
**dadurch gekennzeichnet,**
**daß** mindestens ein Ende des Schneiddrahts (30) über eine Ausgleichsfeder mit dem ersten oder zweiten Element (18, 20) verbunden ist.

10. Vorrichtung nach einem der Ansprüche 2 bis 4,
**dadurch gekennzeichnet,**
**daß** mindestens ein Ende des Schneiddrahts (30) über eine Ausgleichsrolle mit dem ersten oder zweiten Element (18, 20) verbunden ist.

11. Vorrichtung nach einem der vorhergehenden Ansprüche,
**dadurch gekennzeichnet,**
**daß** die Eindringtiefe der Schneidevorrichtung (12) mittels einer Einstellvorrichtung (34) einstellbar ist.

12. Vorrichtung nach einem der vorhergehenden Ansprüche,
**dadurch gekennzeichnet,**
**daß** mindestens ein Schneidelement (28, 30, 32) eine Ultraschallanbindung aufweist.

13. Vorrichtung nach einem der vorhergehenden Ansprüche,
**dadurch gekennzeichnet,**
**daß** mindestens ein Schneidelement (28, 30, 32) mit Strom und/oder Hochfrequenz beaufschlagt ist.

14. Vorrichtung nach einem der vorhergehenden Ansprüche,
**dadurch gekennzeichnet,**
**daß** das oder die Schneidelemente (28, 30, 32) aus einem lichtdurchlässigen Material bestehen.

15. Vorrichtung nach einem der vorhergehenden Ansprüche,
**dadurch gekennzeichnet,**
**daß** innerhalb des ersten hohlförmigen Elements (20) ein erster Unterdruckkanal (36) zum Ansaugen und Fixieren des zu durchtrennenden und zu entnehmenden Gewebes ausgebildet ist.

16. Vorrichtung nach einem der vorhergehenden Ansprüche,
**dadurch gekennzeichnet,**
**daß** die Schneidevorrichtung (12) von einem zweiten Unterdruckkanal zum Ansaugen und Fixieren des zu durchtrennenden Gewebes umgeben ist.

17. Vorrichtung nach einem der vorhergehenden Ansprüche,
**dadurch gekennzeichnet,**
**daß** der Unterdruck im ersten und/oder zweiten Unterdruckkanal (36) mittels einer Vakuumvorrichtung voreinstellbar und/oder regulierbar ist.

18. Vorrichtung nach einem der vorhergehenden Ansprüche,
**dadurch gekennzeichnet,**
**daß** im ersten Unterdruckkanal (36) mindestens ein Sieb (38) oder mindestens eine Filtervorrichtung angeordnet ist.

19. Vorrichtung nach einem der vorhergehenden Ansprüche,
**dadurch gekennzeichnet,**
**daß** innerhalb des ersten hohlförmigen Elements (20) eine Fasersonde zur Einkoppelung eines Lasers, für spektroskopische Messungen, für die optische Kohärenztomographie oder für die konfokale Mikroskopie angeordnet ist.
